# EUROPEAN PATENT APPLICATION

(11) **EP 4 094 761 A1**
(43) Date of publication of application: **30.11.2022**
(21) Application number: 21743663.3
(22) Date of filing: 23.01.2021
(51) Int. Cl.: A61K 31/436, A61K 9/06, A61K 47/10, A61K 47/14, A61P 17/00, A61P 37/06

(54) **EXTERNAL PREPARATION CONTAINING RAPAMYCIN**

(30) Priority: 24.01.2020 JP 2020009633
(71) Applicant: Nobelpharma Co., Ltd., Chuo-ku Tokyo 104-0033 (JP)
(72) Inventor: YASUZAWA, Toru, Tokyo 104-0033 (JP); IGAKI, Matsuo, Tokyo 104-0033 (JP); MATSUMOTO, Takuma, Osaka-shi, Osaka 541-0045 (JP); HIMI, Satoshi, Osaka-shi, Osaka 541-0045 (JP)
(74) Representative: TBK
(86) International application number: PCT/JP2021/002352
(87) International publication number: WO 2021/149827

(57) **Abstract**

To provide a topical formulation that contains rapamycin, has excellent stability, and is effective in treating a skin disease.

A topical formulation including
(a) rapamycin,
(b) at least one selected from the group consisting of ethylene glycol salicylate, diethyl sebacate, and triacetin, and
(c) propylene glycol and/or polyethylene glycol, the topical formulation further including an antioxidant, and the topical formulation further including a solution of an antioxidant in a lipophilic solvent are provided.

## Description

### Technical Field

The present invention relates to a topical formulation containing rapamycin. Specifically, the present invention relates to a topical formulation that contains rapamycin, has excellent stability, and is effective in treating a skin disease.

### Background Art

Rapamycin (generic name: sirolimus) is a macrolide produced by microorganisms. Since rapamycin has an immunosuppressive effect, it has been applied as a therapeutic agent for various diseases so far. For example, in addition to being used as an oral medicine for treating lymphangioleiomyomatosis, rapamycin has more recently been used in a topical formulation for treating a skin lesion associated with tuberous sclerosis.

Meanwhile, although rapamycin is soluble in some organic solvents such as ethanol, it has a property of being almost insoluble or difficult to be dissolved in many solvents such as water.

In a case where rapamycin is used as a topical formulation, it needs to be dispersed in an ointment base or the like by being dissolved in a solvent. As described above, rapamycin has a property of being almost insoluble in many solvents except for some solvents such as ethanol, however, selection of a solvent is required to be performed cautiously, since alcohol has a skin irritating property.

In addition, rapamycin is easily deteriorated by oxidation, and is easily degraded by the presence of water. A medical preparation prepared by combining rapamycin with an antioxidant has thus been developed, however, it has been difficult to disperse a lipid-soluble antioxidant in a non-alcohol-containing solution of rapamycin.

Patent Literature 1 discloses a composition for treating a skin disease, the composition containing rapamycin and polyethylene glycol and not containing alcohol. In addition, Patent Literature 2 discloses an anhydrous composition for treating a skin disease, the composition containing an mTOR inhibitor such as rapamycin, an antioxidant, and a gelling agent. In this patent literature, propylene glycol or the like is used as a solvent.

### Citation List

### Patent Literature

Patent Literature 1: WO 2018/031789 A
Patent Literature 2: WO 2018/129364 A

### Summary of Invention

### Technical Problem

The present invention has been made in view of the above circumstances, and an object of the present invention is to provide a topical formulation containing an effective dose of rapamycin whose stability is secured, the topical formulation not containing alcohol.

### Solution to Problem

The inventors studied various combinations of solvents in which alcohol is not contained, stability of rapamycin can be secured, and an effective dose of rapamycin can be contained, and found that a specific combination of the solvents can solve the above problems, thus completing the present invention.

That is, the present invention is a topical formulation including
(a) rapamycin,
(b) at least one selected from the group consisting of ethylene glycol salicylate, diethyl sebacate, and triacetin, and
(c) propylene glycol and/or polyethylene glycol.

Furthermore, the present invention is a topical formulation including
(a) rapamycin,
(b) at least one selected from the group consisting of ethylene glycol salicylate, diethyl sebacate, and triacetin,
(c) propylene glycol and/or polyethylene glycol, and
(d) an antioxidant.

In addition, the present invention is a topical formulation including
(a) rapamycin,
(b) at least one selected from the group consisting of ethylene glycol salicylate, diethyl sebacate, and triacetin,
(c) propylene glycol and/or polyethylene glycol, and
(d1) a solution of an antioxidant in a lipophilic solvent.

In addition, the present invention is a method for producing a topical formulation, the method including
(i) a step of dissolving (d1) the antioxidant in the lipophilic solvent and
(ii) a step of mixing a solution obtained in (i) with a solution containing the following (a), (b), and (c).
   (a) rapamycin
   (b) at least one selected from the group consisting of ethylene glycol salicylate, diethyl sebacate, and triacetin
   (c) propylene glycol and/or polyethylene glycol

The present invention is a topical formulation obtained by mixing
(i) a mixture obtained by dissolving an antioxidant in a lipophilic solvent with
(ii) a solution containing the following (a), (b), and (c).
   (a) rapamycin
   (b) at least one selected from the group consisting of ethylene glycol salicylate, diethyl sebacate, and triacetin
   (c) propylene glycol and/or polyethylene glycol

### Advantageous Effects of Invention

The present invention can provide a highly stable preparation, specifically, a topical formulation, in which rapamycin is stably present without being precipitated during the storage of the preparation, and degradation of rapamycin in the preparation is suppressed.

### Brief Description of Drawings

FIG. 1A illustrates stability (residual ratio) of sirolimus at 40°C in an aqueous ethanol solution of sirolimus and various sirolimus-BHT mixture solutions.
FIG. 1B illustrates stability (residual ratio) of sirolimus at 50°C in an aqueous ethanol solution of sirolimus and various sirolimus-BHT mixture solutions.
FIG. 2 illustrates stability (residual ratio) of sirolimus at 40°C or 50°C when BHT is mixed at concentrations of 1% to 10%.

### Description of Embodiments

Topical formulation containing rapamycin

A topical formulation of the present invention includes
(a) rapamycin,
(b) at least one selected from the group consisting of ethylene glycol salicylate, diethyl sebacate, and triacetin, and
(c) propylene glycol and/or polyethylene glycol.

(a) Rapamycin is known under a generic name sirolimus, and commercially available rapamycin can be used. In the present specification, rapamycin and sirolimus are used as synonyms.

Note that, instead of rapamycin, everolimus or temsirolimus known as a derivative of rapamycin can be used in place of rapamycin or in combination with rapamycin.

The amount of rapamycin used can be 0.05 to 1 weight%, preferably 0.1 to 0.8%, and particularly preferably 0.1 to 0.5%, with respect to the entire preparation.

As the solvent (b), at least one selected from the group consisting of ethylene glycol salicylate, diethyl sebacate, and triacetin may be used, or these solvents may be used by being mixed at an arbitrary ratio.

As the solvent (c), propylene glycol or polyethylene glycol, or a mixture thereof may be used. A mixture of propylene glycol and polyethylene glycol (Macrogol 400) is particularly preferable, and these may be used by being mixed at an arbitrary ratio.

A mixing ratio between the solvent (b) and the solvent (c) is not particularly limited, and examples thereof include (b):(c) = 2:1 to 2:6 as a weight ratio. (b):(c) = 2:5 is particularly preferably suggested as the mixing ratio.

Meanwhile, a mixing ratio between (a) rapamycin and the solvent (c) is not limited as long as rapamycin dissolves in a mixed solution of the solvent (b) and the solvent (c). Examples of the mixing ratio include (a):(c) = 1:20 to 1:70 as a weight ratio, and the mixing ratio is preferably (a):(c) = 1:60. At a weight ratio including the solvent (b), the mixing ratio is (a):(b):(c) = 1:20 to 80:20 to 70 and preferably (a):(b):(c) = 1:25:60.

An antioxidant (d) can be further added to the topical formulation of the present invention. By adding the antioxidant to the topical formulation, degradation of rapamycin can be prevented, thus ensuring long-term storage stability. Specific examples of the antioxidant include dibutylhydroxytoluene (BHT), tocopherol, and ascorbic acid, and dibutylhydroxytoluene (BHT) is preferably suggested as the antioxidant.

Examples of the amount of the antioxidant used include 1 to 10 weight% with respect to the entire preparation, and preferably 1 to 5% and particularly preferably 1 to 2% is suggested as the amount.

The antioxidant can be directly added to the preparation containing (a), (b), and (c) above, however, it is preferable that a solution (d1) obtained by dissolving the antioxidant in a lipophilic solvent is added to the preparation containing (a), (b), and (c) above.

Examples of such lipophilic solvent include liquid paraffin and a mixture of liquid paraffin and polyethylene (hydrocarbon gel), and liquid paraffin is preferably suggested as the lipophilic solvent.

Examples of the amount of the lipophilic solvent used with respect to the antioxidant include antioxidant:lipophilic solvent = 1:10 to 3:10 as a weight ratio, and 3:10 is preferably suggested as the amount.

A surfactant can be further added to the solution (d1) obtained by dissolving the antioxidant in the lipophilic solvent. Preferable examples of the surfactant include a surfactant with an HLB of 4 to 7, and one or more surfactants with an HLB of 5 or 6 are preferable.

The obtained preparation is further stabilized by adding such surfactant.

Examples of the surfactant include at least one, preferably, at least two, selected from the group consisting of polyoxyethylene cetyl ether, glyceryl monostearate, and polyoxyethylene hydrogenated castor oil.

Specific examples of the surfactant include
- POE cetyl ether:glyceryl monostearate = 1:4 (weight ratio) and
- glyceryl monostearate:POE hydrogenated castor oil = 4:1 (weight ratio).

The topical formulation of the present invention may also contain an additional excipient or additive that is generally used within the scope that does not impair the effects of the invention, in order to obtain the topical formulation in a drug dosage form. For example, as such excipient or additive, a gelling agent, a thickening agent, a pH adjuster, inorganic salts, or the like may be applied.

Examples of the gelling agent include a polymer derived from water-soluble cellulose, for example, hydroxymethyl cellulose, hydroxyethyl cellulose, hydroxypropyl cellulose, and hydroxypropyl methyl cellulose, carboxymethyl cellulose, methyl hydroxyethyl cellulose, methyl cellulose, and carrageenan. Examples of the thickening agent include hydroxypropyl cellulose. Examples of the pH adjuster include an acidic additive such as hydrochloric acid, sulfuric acid, phosphoric acid, citric acid, tartaric acid, malic acid, mesylic acid, tosic acid, and besylic acid and a buffer containing an alkali metal salt, an alkaline earth metal salt, or an ammonium salt. Furthermore, examples of the inorganic salts include calcium chloride, sodium chloride, calcium oxide, and magnesium sulfate.

Additional medicinal component can be further added to the topical formulation of the present invention, as long as it does not impair the effects of the main active ingredient, rapamycin. Examples of such additional medicinal component include tacrolimus and steroids.

### Method for producing topical formulation

The topical formulation of the present invention can be produced in the following manner.

For example,
(a) rapamycin,
(b) at least one selected from the group consisting of ethylene glycol salicylate, diethyl sebacate, and triacetin, and
(c) propylene glycol and/or polyethylene glycol, are mixed with each other.

In addition, in a case of using the antioxidant,
(i) a step of dissolving (d1) the antioxidant in the lipophilic solvent and
(ii) a solution obtained in (i) is mixed with a solution containing the following (a), (b), and (c).
   (a) rapamycin
   (b) at least one selected from the group consisting of ethylene glycol salicylate, diethyl sebacate, and triacetin
   (c) propylene glycol and/or polyethylene glycol

In addition, in order to obtain the topical formulation with higher stability, the topical formulation may be produced by, for example,
(i) (d2) a step of preparing a mixed solution by dissolving an antioxidant in a lipophilic solvent and then adding one or more surfactants with an HLB of 5 to 7 to the mixed solution and
(ii) a step of mixing a solution obtained in (i) with a solution containing the following (hereinafter, may be referred to as a "solution of rapamycin in a hydrophilic solvent")
   (a) rapamycin,
   (b) at least one selected from the group consisting of ethylene glycol salicylate, diethyl sebacate, and triacetin, and
   (c) propylene glycol and/or polyethylene glycol.

In particular, by adding one or more surfactants with an HLB of 5 to 7 to the mixed solution obtained by dissolving the antioxidant in the lipophilic solvent in the step (d2) in (i) above, compatibility between the mixed solution of the antioxidant and the lipophilic solvent and the solution of rapamycin in a hydrophilic solvent in the step of (ii) above can be further increased.

In order to obtain the topical formulation of the present invention in an optimal drug dosage form, for example, forms such as a cream, a paste, a jelly, a gel, an emulsion, or a liquid, for example, an ointment, a liniment, or a lotion, aside from the additional excipients or additives described above, one or more selected from sodium alginate; a polymer such as gelatin, corn starch, gum tragacanth, methyl cellulose, hydroxyethyl cellulose, carboxymethyl cellulose, xanthan gum, dextrin, carboxymethyl starch, polyvinyl alcohol, sodium polyacrylate, a methoxyethylene-maleic anhydride copolymer, polyvinyl ether, and polyvinylpyrrolidone; beeswax, olive oil, cacao oil, sesame oil, soybean oil, Camellia oil, peanut oil, beef tallow, lard, and lanolin; petrolatum such as white petrolatum and yellow petrolatum; paraffin and a mixture of liquid paraffin and polyethylene (hydrocarbon gel); stearic acid; lauric acid ester, myristic acid ester, and octanoic acid ester; cetyl alcohol and stearyl alcohol; polyethylene glycol; dimethyl sulfoxide and dodecylpyrrolidone; urea; Azone; olive oil; and kaolin, bentonite, zinc oxide, and titanium oxide can be suitably added to the topical formulation.

### Method for using topical formulation

The topical formulation of the present invention containing rapamycin can be used for treating, for example, a skin disease, since the topical formulation has an immunosuppressive effect.

Specific examples of the skin disease include dermatitis, contact dermatitis, psoriasis, atopic dermatitis, seborrheic dermatitis, nummular eczema, vitiligo, rosacea, keloid, autosensitization dermatitis, stasis dermatitis, asteatotic eczema, a skin tumor in tuberous sclerosis, seborrheic keratosis, and a skin tumor in Recklinghausen's disease.

A dose of a pharmaceutical product obtained using the topical formulation of the present invention can be naturally changed depending on the sex, age, physiological condition, pathological condition, or the like of a patient. For example, in a case of using as a topical formulation, administration can be performed by applying 0.01 to 100 mg/m² (body surface area) of rapamycin to, for example, an adult per day.

### Examples

Hereinafter, the present invention will be described with reference to specific embodiments, however, the present invention is not limited to these embodiments, and it should be understood that various changes and modifications can be made therein by those skilled in the art without departing from the scope and spirit of the present invention as defined in the appended claims.

### Reference Example 1

Sirolimus was mixed into each solvent shown in Table 1 so that sirolimus:solvent was a ratio of 1:50 (weight ratio), heating and ultrasonic irradiation were performed thereon in a water bath of about 50°C, and solubility was confirmed by visual observation. As a result, as shown in Table 1, it was confirmed that sirolimus did not dissolve in a single solvent.

### [Table 1]

**(Table 1)**

| Solubility of sirolimus in each solvent | |
|---|---|
| Solvent | Solubility |
| Medium-chain fatty acid triglyceride | Insoluble (cloudy liquid) |
| Ethylene glycol salicylate | Insoluble (cloudy liquid) |
| Diethyl sebacate | Insoluble (cloudy liquid) |
| Diisopropyl sebacate | Insoluble (cloudy liquid) |
| Triacetin | Insoluble (cloudy liquid) |
| Squalane | Insoluble (cloudy liquid) |
| Propylene glycol | Insoluble (cloudy liquid) |
| Polyethylene glycol (Macrogol 400) | Insoluble (cloudy liquid) |
| 1,3 Butylene glycol | Insoluble (cloudy liquid) |

### (Quantitative test for sirolimus)

Quantification of sirolimus was performed by conducting an HPLC test under the following conditions and comparing the area of a sirolimus peak in a test substance and the area of a sirolimus peak in a sirolimus standard.

Detector: ultraviolet absorption photometer (detection wavelength: 278 nm)
Column: stainless steel tube with internal diameter of 4.6 mm and length of 250 mm filled with octadecylsilyl-silica gel for chromatography
Column temperature: constant temperature of about 40°C
Mobile phase: acetonitrile/0.02 mol/L ammonium acetate buffer (pH 6.0) = 13/7
Flow rate: 0.8 mL/min

### Example 1 and Comparative Example 1

Various preparations were prepared by using mixed solvents obtained by mixing each of the solvents indicated in Table 1 with propylene glycol, mixing sirolimus with the mixed solvents so that sirolimus:solvent :propylene glycol was 1:50:25 (weight ratio), and performing heating and ultrasonic irradiation thereon in a water bath of about 50°C. The obtained preparations were visually observed to confirm solubility.

Results of the solubility are shown in Table 2. Dissolution of sirolimus was confirmed when mixed solutions of ethylene glycol salicylate, diethyl sebacate, and triacetin and propylene glycol were used.

### [Table 2]

**(Table 2)**

| Solubility of sirolimus in mixed solution of each solvent and propylene glycol (PG) | | |
|---|---|---|
| Test Example | Solvent | Solubility |
| Comparative Example 1-1 | Medium-chain fatty acid triglyceride + PG | Insoluble (precipitation of white substance) |
| Example 1-1 | Ethylene glycol salicylate + PG | Soluble |
| Example 1-2 | Diethyl sebacate + PG | Soluble |
| Comparative Example 1-2 | Diisopropyl sebacate + PG | Insoluble (cloudy liquid) |
| Example 1-3 | Triacetin + PG | Soluble |
| Comparative Example 1-3 | Squalane + PG | Insoluble (separation into two phases) |
| Comparative Example 1-4 | Polyethylene glycol (Macrogol 400) + PG | Insoluble (cloudy liquid) |
| Comparative Example 1-5 | 1,3 Butylene glycol + PG | Insoluble (cloudy liquid) |

Note that the same effects are also obtained by preparing the topical formulation using polyethylene glycol instead of propylene glycol.

### Reference Examples 2 and 3 (examination of mixing of antioxidant)

A solution obtained by dissolving sirolimus in a 50% aqueous ethanol solution at a ratio of 0.2% (hereinafter, referred to as an aqueous ethanol solution of sirolimus) and a solution obtained by mixing BHT into the aqueous ethanol solution of sirolimus at a ratio of 0.1% (hereinafter, referred to as a sirolimus-BHT mixture solution) were prepared. Furthermore, a solution was prepared by adding sodium EDTA or sodium tocopheryl acetate to the sirolimus-BHT mixture solution. Each of the ethanol solution of sirolimus and various sirolimus-BHT mixture solutions was stored at 40°C and 50°C, and, after 7 hours and 30 hours, quantification was performed on samples thereof by the method described in the section of "Quantification test" above to calculate residual ratios of sirolimus.

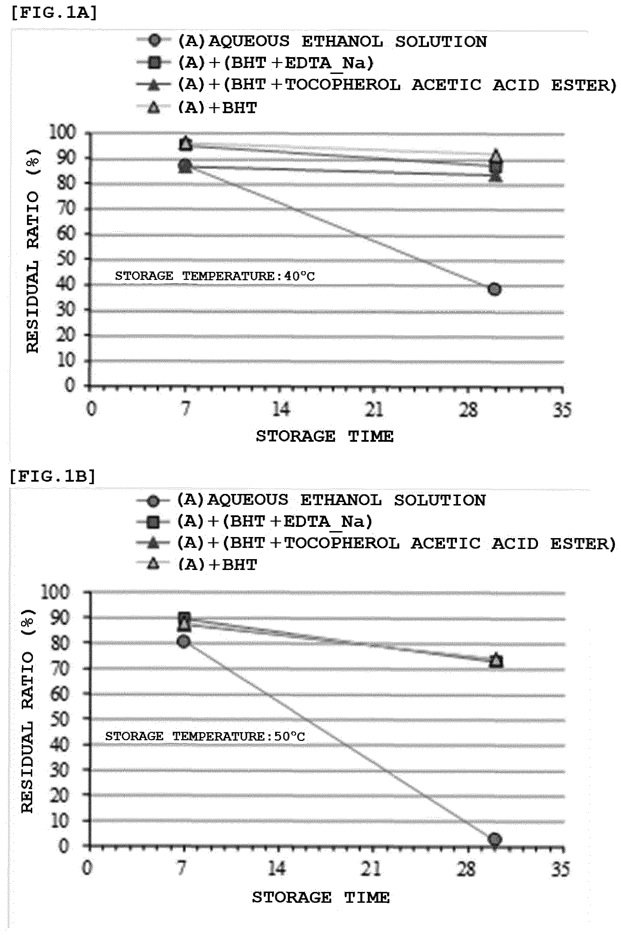

Compositions of the sample solutions are shown in Table 3, and the results are shown in Table 4 and FIGS. 1A and 1B. The residual ratios decreased over time in the aqueous ethanol solution of sirolimus at both temperatures (Reference Example 2).

On the other hand, decreases in the residual ratios were predominantly suppressed in the various sirolimus-BHT mixture solutions at both temperatures (Reference Example 3) .

### [Table 3]

**(Table 3)**

| Prescription for examining antioxidant | | | | |
|---|---|---|---|---|
| Name of component | (A) | (A) + BHT + EDTA | (A) + BHT + tocopherol | (A) + BHT |
| | Control for comparison (No. 6-0) | Examination-1 (No. 6-1) | Examination-2 (No. 6-2) | Effects of BHT (No. 3-4-2) |
| Sirolimus | 0.2 g | 0.20 g | 0.20 g | 0.20 g |
| Ethanol | 49.42 g | 49.42 g | 49.42 g | 49.90 g |
| BHT | - | 0.10 g | 0.10 g | 0.10 g |
| EDTA-Na | - | 0.02 g | - | - |
| Tocopherol acetic acid ester | - | - | 0.10 g | - g |
| Purified water | 50.38 g | 50.26 g | 50.18 g | 50.00 g |
| Total | 100.0 g | 100.0 g | 100.0 g | 100.2 g |

### [Table 4]

**(Table 4)**

| Effects of mixing BHT | | | | |
|---|---|---|---|---|
| | 40°C | | 50°C | |
| | 7 | 30 | 7 | 30 |
| (A) Aqueous ethanol solution | 87.3 | 38.4 | 80.6 | 2.7 |
| (A) + (BHT + EDTA Na) | 95.4 | 87.4 | 89.7 | 73.0 |
| (A) + (BHT + tocopherol acetic acid ester) | 87.0 | 84.0 | 87.2 | 73.8 |
| (A) + BHT | 96.3 | 92.0 | 88.1 | 74.0 |

### Reference Example 4 (examination of mixing amount of BHT)

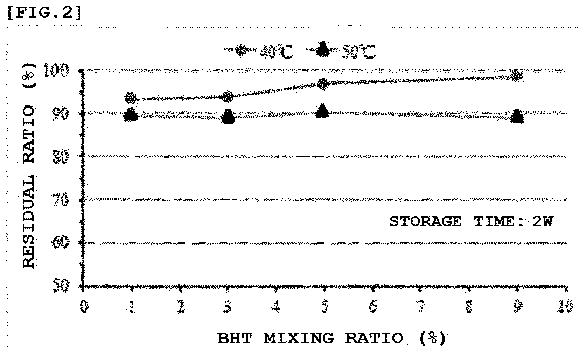

Solutions were prepared by mixing various concentrations of BHT into the ethanol solution of sirolimus, and the solutions were stored at 40°C and 50°C for 2 weeks. Quantification of sirolimus was performed on samples after the storage by the same method as that in Reference Example 2 to calculate residual ratios. The results are shown in Table 5 and FIG. 2. It was confirmed that stability of sirolimus was maintained when BHT was mixed at a concentration of 1% or higher.

### [Table 5]

**(Table 5)**

| Effects of mixing amount of BHT on stability of sirolimus | | | | | |
|---|---|---|---|---|---|
| Mixing amount (with respect to indicated amount, %) | Initial amount (%) | 40°C 2W | | 50°C 2W | |
| | | Quantified value (%) | Residual ratio (%) | Quantified value (%) | Residual ratio (%) |
| 1 | 97.34 | 90.86 | 93.3 | 87.15 | 89.5 |
| 3 | 96.73 | 90.67 | 93.7 | 85.91 | 88.8 |
| 5 | 98.06 | 94.89 | 96.8 | 88.42 | 90.2 |
| 9 | 95.65 | 94.25 | 98.5 | 84.91 | 88.8 |

### Example 2 (mixing of thickened sirolimus solution with BHT solution)

A solution was prepared by dissolving sirolimus in a mixed solution of triacetin and propylene glycol (5:7) at a ratio of 1.6%. The solution was thickened by adding hydroxypropyl cellulose thereto at a ratio of 1.6 weight% (hereinafter, referred to as a thickened sirolimus solution).

A separate solution was prepared by dissolving BHT in liquid paraffin at a concentration of 23% (hereinafter, referred to as a BHT solution).

The thickened sirolimus solution and the BHT solution were mixed with each other at a ratio of 70:30, and high-speed stirring was performed under a condition of 11,000 min⁻¹. The obtained solution was allowed to stand for 3 hours and visually observed. As a result, the solution which appeared to be homogeneously mixed immediately after the stirring was gradually separated into two phases.

### Example 3 (mixing of thickened sirolimus solution with BHT/surfactant solution)

Various substances known as surfactants were mixed at ratios indicated in Table 6 to prepare surfactants with various HLB values.

Solutions obtained by adding the various prepared surfactants to the BHT solution at a ratio of 13% while heating (50 to 60°C) were mixed with the thickened sirolimus solution at a ratio of 62:38, and high-speed stirring was performed under a condition of 11,000 min-1, thus obtaining preparations. The obtained preparations were then allowed to stand for 22 hours, and the states of the solutions were visually observed.

As a result of the visual observation, in the preparations obtained using the surfactants a and b, phase separation did not occur, and cloudy mixed states were stably maintained. On the other hand, phase separation occurred in the preparations obtained using the surfactants with the HLB values of 7.25 or higher. The results indicate that it is preferable to use a surfactant with an HLB value of 5 to 7 in order to suppress phase separation.

### [Table 6]

**(Table 6)**

| Prepared surfactants | | | | | |
|---|---|---|---|---|---|
| Surfactant (HLB value of surfactant alone) | Sample No./Mixing ratio | | | | |
| | a | b | c | d | e |
| POE cetyl ether (10.5) | 0.2 | | 0.5 | 0.8 | |
| Glyceryl monostearate (4.0) | 0.8 | 0.8 | 0.5 | 0.2 | |
| POE hydrogenated castor oil (14.0) | | 0.2 | | | |
| Polysorbate 60 (14.9) | | | | | 1.0 |
| HLB value after preparation | 5.3 | 6.0 | 7.25 | 9.2 | 14.9 |

### Example 4 and Comparative Example 2

Ointments were prepared by the following procedures and stored at 50°C, and residual ratios of sirolimus were measured after 2 weeks and 4 weeks.

### (Example 4)

A solution obtained by dissolving rapamycin in a triacetin/propylene glycol/polyethylene glycol mixed solution (a solution obtained by mixing triacetin, propylene glycol, and polyethylene glycol at a ratio of approximately equal amounts) was added to a hydrocarbon gel, so that a ratio of rapamycin was 0.2% with respect to a final agent. This mixture was mixed with a liquid paraffin solution of BHT in the presence of a surfactant (a mixed solution of glyceryl monostearate and polyoxyethylene hydrogenated castor oil, mixed so that HLB = about 6), so that a ratio of BHT was 1.5% with respect to the final agent. When the content of the hydrocarbon gel was adjusted to be about 75% of the final agent, a gel-like ointment was obtained.

### (Comparative Example 2)

A solution obtained by dissolving rapamycin in a triacetin/propylene glycol mixed solution was added to a hydrocarbon gel, so that a ratio of rapamycin was 0.2% with respect to a final agent. When the content of the hydrocarbon gel was adjusted to be about 80% of the final agent, a gel-like ointment was obtained.

The results are shown in Table 7. From the table, it is found that a decrease in the residual amount of sirolimus was suppressed, and sirolimus was stably maintained in the ointment obtained by mixing in BHT (Example 4), whereas the residual amount of sirolimus rapidly decreased with the lapse of storage time in the ointment obtained without mixing in BHT (Comparative Example 2).

### [Table 7]

**(Table 7)**

| Stability of sirolimus ointment | | | |
|---|---|---|---|
| | Initial amount % | After 2 weeks % | After 4 weeks % |
| Comparative Example 2 | 100 | 55.1 | 9.2 |
| Example 4 | 100 | 97.3 | 97.4 |

### Example 5

### Penetration test for ointment

Penetration of an ointment into a skin model was tested using the sirolimus ointment prepared in Example 4 as a test substance and an EFT-400 kit manufactured by MetTec Corporation as the skin model.

Skin model cells were transferred to a 6-well plate into which an EFT-400 assay medium was dispensed, and the cells were incubated overnight (for 16 to 18 hours) in a carbon dioxide incubator (37°C, 5% CO2, under a humidified condition).

About 50 mg of the test substance was applied onto the center of the cultured skin cells and evenly spread on the cells. These cells were incubated for 24 hours in a carbon dioxide incubator (37°C, 5% CO₂, under a humidified condition). After completing the incubation, the test substance on the cells was wiped off 3 times using absorbent cotton moistened with phosphate buffered saline, and then moisture on the surface was removed. The dermic layer was peeled off, and the surface thereof was washed with phosphate buffered saline. Then, moisture on the surface was removed to measure the mass.

The collected dermic layer was cut into small pieces, 1 mL of methanol was added thereto, and the mixture was vigorously stirred. The mixture was subjected to centrifugation (20,400 × g, 5 minutes, 4°C), and the supernatant was used as a sample solution. Furthermore, a solution prepared by performing the same operation as above without applying the test substance was used as a blank matrix. A methanol solution of ascomycin (20 ng/mL) was separately prepared and used as an internal standard solution.

Then, the sample solution was diluted 10-fold with the blank matrix, and 20 µL of the diluted sample solution was collected in a PP tube. 20 µL of acetonitrile, 20 µL of the internal standard solution, and 50 µL methanol were added to and mixed with the diluted sample solution, and the mixture was subjected to LC/MS/MS under the following conditions to quantify a sirolimus concentration in the measurement sample. The mass of sirolimus that transferred to the dermic layer was calculated from the obtained value of the sirolimus concentration and the mass of the dermic layer used in the preparation of the sample solution. A rate of transfer to the dermic layer (% with respect to the indicated dose) was obtained from the mass of the sirolimus transferred to the dermic layer and the mass of sirolimus in the applied test substance. As a result, the rate of sirolimus transferred to the dermic layer was 1.402 (% with respect to the indicated dose), and it was confirmed that skin penetrating properties were sufficiently maintained.

(LC conditions)
Column: stainless steel tube with internal diameter of 2.0 mm and length of 50 mm filled with octadecylsilyl-silica gel for chromatography
Column temperature: constant temperature of about 50°C
Flow rate: 0.6 mL/min
Mobile phase: 5 mmol/L ammonium formate (liquid A) and methanol (liquid B) mixed under the following gradient conditions

### [Table 8]

**(Table 8)**

| Gradient conditions | | |
|---|---|---|
| Time (min) | Liquid A (%) | Liquid B (%) |
| initial | 20.0 | 80.0 |
| 0.80 | 20.0 | 80.0 |
| 0.81 | 10.0 | 90.0 |
| 1.10 | 10.0 | 90.0 |
| 1.11 | 20.0 | 80.0 |
| 1.40 | 20.0 | 80.0 |

(MS/MS conditions)
Ionization method: ESI
Polarity: positive
Scan type: multiple reaction monitoring (MRM)
Confirmed ion species (m/z, Q1>Q3): sirolimus (931>864): ascomycin (809>756)

### Industrial Applicability

According to the present invention, a stable topical formulation can be provided, in which solubility of rapamycin is excellent and which has a high rate of transfer to a dermic layer. Therefore, according to the present invention, the treatment of a skin disease with rapamycin can be performed more broadly and more effectively.

## Claims

1. A topical formulation comprising:
(a) rapamycin;
(b) at least one selected from the group consisting of ethylene glycol salicylate, diethyl sebacate, and triacetin; and
(c) propylene glycol and/or polyethylene glycol.

2. A topical formulation comprising:
(a) rapamycin;
(b) at least one selected from the group consisting of ethylene glycol salicylate, diethyl sebacate, and triacetin;
(c) propylene glycol and/or polyethylene glycol; and
(d) an antioxidant.

3. A topical formulation comprising:
(a) rapamycin;
(b) at least one selected from the group consisting of ethylene glycol salicylate, diethyl sebacate, and triacetin;
(c) propylene glycol and/or polyethylene glycol; and
(d1) a solution of an antioxidant in a lipophilic solvent.

4. The topical formulation according to any one of claims 1 to 3, wherein a weight ratio between (a) and (c) is 1:20 to 1:70.

5. The topical formulation according to any one of claims 1 to 3, wherein a weight ratio between (b) and (c) is 2:1 to 2:6.

6. The topical formulation according to any one of claims 1 to 3, wherein a weight ratio between (a), (b), and (c) is 1:20 to 80:20 to 70.

7. The topical formulation according to claim 2 or 3, wherein the antioxidant is dibutylhydroxytoluene (BHT).

8. The topical formulation according to claim 3, wherein the solution of the antioxidant in the lipophilic solvent in (d1) further contains one or more surfactants with an HLB of 5 to 7.

9. A method for producing the topical formulation according to claim 3, the method comprising:
(i) a step of dissolving (d1) the antioxidant in the lipophilic solvent; and
(ii) a step of mixing a solution obtained in (i) with a solution containing the following (a), (b), and (c),
(a) rapamycin,
(b) at least one selected from the group consisting of ethylene glycol salicylate, diethyl sebacate, and triacetin
(c) propylene glycol and/or polyethylene glycol.

10. The method for producing the topical formulation according to claim 9, wherein the step (i) further includes mixing in one or more surfactants with an HLB of 5 to 7.

11. The method for producing the topical formulation according to claim 10, wherein one or more surfactants with an HLB of 5 to 7 are at least two selected from the group consisting of polyoxyethylene cetyl ether, glyceryl monostearate, and polyoxyethylene hydrogenated castor oil.

12. A topical formulation obtained by mixing
(i) a mixture obtained by dissolving an antioxidant in a lipophilic solvent with
(ii) a solution containing the following (a), (b), and (c),
(a) rapamycin,
(b) at least one selected from the group consisting of ethylene glycol salicylate, diethyl sebacate, and triacetin, and
(c) propylene glycol and/or polyethylene glycol.

13. The topical formulation according to claim 12, wherein the mixture in (i) further contains one or more surfactants with an HLB of 5 to 7.
